# DEMANDE DE BREVET EUROPEEN

(11) **EP 2 298 297 A1**
(43) Date de publication de la demande: **23.03.2011**
(21) Numéro de dépôt: 10011132.7
(22) Date de dépôt: 20.12.2006
(51) Int. Cl.: A61K 31/4045, A61K 31/55, A61K 45/06, A61P 9/12

(54) **Association d'un inhibiteur du courant if sinusal et d'un inhibiteur de l'enzyme de conversion**

(30) Priorité: 21.12.2005 FR 0513006
(62) Demande divisionnaire de: 06291993.1
(71) Demandeur: Les Laboratoires Servier, 92284 Suresnes Cedex (FR)
(72) Inventeur: Benatar, Vidal, 92210 Saint-Cloud (FR); Lerebours-Pigeonnière, 92300 Levallois-Perret (FR)
(74) Mandataire: Bestel, Delphine

(57) **Abrégé**

Association contenant un inhibiteur sélectif et spécifique du courant I_{f} sinusal et plus particulièrement l'ivabradine et un agent inhibiteur de l'enzyme de conversion de l'angiotensine.

## Description

La présente invention concerne une nouvelle association d'un inhibiteur sélectif et spécifique du courant I_{f} sinusal et d'un agent inhibiteur de l'enzyme de conversion de l'angiotensine.

Plus particulièrement, la présente Invention concerne une nouvelle association d'un inhibiteur sélectif et spécifique du courant I_{f} sinusal qui est l'ivabradine ou 3-{3-[{[(7S)-3,4-diméthoxybicyclo[4.2.0]octa-1,3,5-trién-7-yl]méthyl}(méthyl)amino]propyl}-7,8-di-méthoxy-1,3,4,5-tétrahydro-2*H*-3-benzazépin-2-one de formule (I): 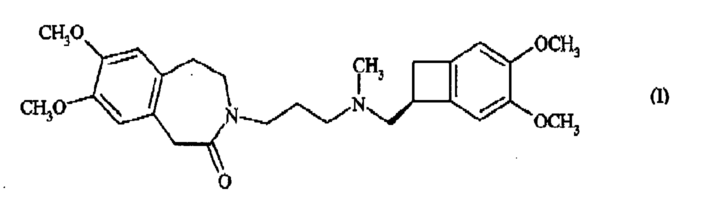 ainsi que ses hydrates, formes cristallines et sels d'addition à un acide pharmaceutiquement acceptable, et d'un agent inhibiteur de l'enzyme de conversion de l'angiotensine.

Les inhibiteurs sélectifs et spécifiques du courant I_{f} sinusal et plus particulièrement l'ivabradine, ainsi que ses hydrates, formes cristallines et ses sels d'addition à un acide pharmaceutiquement acceptable, et plus particulièrement son chlorhydrate, possèdent des propriétés pharmacologiques et thérapeutiques très intéressantes, notamment des propriétés chronotropes négatives (réduction de la fréquence cardiaque), qui rendent ces composés utiles dans le traitement, la prévention et l'amélioration du pronostic de différentes maladies cardio-vasculaires liées à l'ischémie myocardique telles que l'angine de poitrine, infarctus du myocarde et les troubles du rythme associés, ainsi que dans les différentes pathologies comportant des troubles du rythme, notamment supra-ventriculâires, et dans l'insuffisance cardiaque chronique.

La préparation et l'utilisation en thérapeutique de l'ivabradine et de ses sels d'addition à un acide pharmaceutiquement acceptable, et plus particulièrement de son chlorhydrate; ont été décrits dans le brevet européen EP 0534 859.

La demanderesse a présentement découvert que, de façon surprenante, les inhibiteurs sélectifs et spécifiques du courant I_{f} sinusal et plus particulièrement l'ivabradine ou 3-{3-[{[(7S)-3,4-dimémoxybicyclo[4.2.0]octa-1,3,5-trién-7-yl]méthyl}(méthyl)ammo]propyl}-7,8-diméthoxy-1,3,4,5-tétrahydro-2*H*-3-benzazépin-2-one, utilisés en association avec un agent inhibiteur de l'enzyme de conversion de l'angiotensine, possédaient des propriétés agent inhibiteur de l'enzyme de conversion de l'angiotensine, possédaient des propriétés intéressantes permettant leur utilisation dans le traitement de l'hypertension artérielle.

L'hypertension artérielle est une maladie silencieuse, mais insidieuse : si, la plupart du tempes, elle ne s'accompagne d'aucun trouble immédiat, elle se manifeste - quand elle n'est pas traitée - au bout de 10 à 20 ans, par la survenue d'un grave accident vasculaire, cardiaque ou cérébral. Au-delà d'un paramètre biologique défini par le dépassement d'une norme, fixée par des experts, l'hypertension artérielle constitue un facteur de risque majeur des maladies cardiovasculaires - qui représentent la première cause de mortalité du dernier tiers de la vie humaine, dans les pays industrialisés.

Cette "bombe à retardement" sanitaire, pose donc un très vaste problème de santé publique, compte tenu de sa fréquence élevée parmi la population générale. Sur le plan thérapeutique, les mesures hygieno-diététiques sont toujours nécessaires. Elles permettent d'éviter parfois, mais le plus souvent simplement de retarder la mise en route d'un traitement médicamenteux. L'arsenal médicamenteux est large mais reste souvent insuffisant pour obtenir un contrôle satisfaisant de la pression artérielle.

On sait que le niveau de pression artérielle (systolique ou diastolique) est un déterminant très important du risque de survenue d'un accident vasculaire cérébral ou d'un infarctus du myocarde. De très nombreux essais cliniques ont démontré que la réduction de la pression artérielle diminuait de façon très significative le risque d'accidents cérébraux et cardiaques chez les hypertendus.

Il est connu que la réponse individuelle à un traitement antihypertenseur est variable et difficilement prévisible. L'obtention d'un contrôle optimal de la pression artérielle nécessite le recours à une plurithérapie (avec des médicaments de mécanisme d'action différents) chez la plupart des patients hypertendus.

On recherche toujours des molécules actives sur les formes d'hypertension qui résistent aux traitements actuels. Par ailleurs, on recherche également des médicament à plus longue durée d'action, ou ayant encore moins d'effets secondaires cliniques ou biologiques.

Ainsi, l'élaboration de nouveaux traitements alternatifs est toujours d'actualité et reste une nécessité.

Une classe thérapeutique largement utilisée dans le traitement de l'hypertension artérielle est constituée par les inhibiteurs de l'enzyme de conversion de l'angiotensine (IEC).

Les inhibiteurs de l'enzyme de conversion sont une des classes thérapeutiques majeures dans le traitement de l'hypertension artérielle. Ils agissent principalement par l'inhibition de la synthèse de l'angiotensine II et par un blocage de la dégradation de la bradykinine. Leurs effets hémodynamiques consistent essentiellement en la diminution des résistances périphériques totales par vasodilatation artériolaire, ce qui entraîne une baisse de la pression artérielle sans stimulation sympathique ni rétention hydrosodée. Ils sont efficace dans tous les types d'hypertension artérielle. Ils ont montré qu'an delà de la baisse de la pression artérielle ils amélioraient la morbidité (infarctus du myocarde, accidents vasculaires cérébraux) et la mortalité cardiovasculaire des hypertendus, des diabétiques, des malades avec une maladie coronaire préexistante.

Ils sont en général très bien tolérés à part l'apparition chez certains patients: d'une toux sèche, réversible à l'arrêt du traitement.

La demanderesse a présentement découvert que, de façon surprenante, les inhibiteurs sélectifs et spécifiques du courant I_{f} sinusal et plus particulièrement l'ivabradine étaient capables de potentialiser les effets des agents inhibiteurs de l'enzyme de conversion de l'angiotensine. Cet effet, non prévisible du fait même de la classe thérapeutique des inhibiteurs sélectifs et spécifiques du courant I_{f} sinusal, permet d'envisager l'utilisation de l'association selon l'invention dans le traitement de l'hypertension artérielle, et plus particulièrement cette potentialisation des effets permettra d'utiliser l'association selon l'invention dans le traitement de patients non contrôlés par les associations thérapeutiques classiques.

Les IEC utilisables dans l'association selon l'invention sont, à titre non limitatif : le Perindopril, le Captopril ,l'Enalapril, le Lisinopril, le Delapril, le Fosinopril, le Quinapril, le Ramipril, le Spirapril, l'Imidapril, le Trandolapril, le Benazepril, le Cilazapril et le Temocapril, ainsi que leurs hydrates, formes cristallines et sels d'addition à un acide ou à une base pharmaceutiquement acceptable.

Les IEC préférés sont le Perindopril, le Captopril, l'Enalapril, le Ramipril, le Lisinopril, le Benazapril, le Quinapril et le Delapril ainsi que leurs hydrates, formes cristallines et sels d'addition à un acide ou à une base pharmaceutiquement acceptable, et plus spécialement le Perindopril ou un de ses hydrates, formes cristallines et sels d'addition à un acide ou à une base pharmaceutiquement acceptable, et plus particulièrement ses sels de tert-butylaminé ou d'arginine.

Les inhibiteurs sélectifs et spécifiques du courant I_{f} sinusal sont l'ivabradine et YM758. de chez Astellas ainsi que leurs hydrates, formes cristallines et sels d'addition à un acide ou à une base pharmaceutiquement acceptable.

Plus particulièrement, l'invention concerne l'association de l'ivabradine ou un de ses hydrates, formes cristallines, et sels d'addition à un acide pharmaceutiquement acceptable et plus particulièrement son chlorhydrate, et d'un agent inhibiteur de l'enzyme de conversion de l'angiotensine ou un de ses hydrates, formes cristallines, et sels d'addition à un acide pharmaceutiquement acceptable.

Plus particulièrement, l'invention concerne l'association entre l'ivabradine ou un de ses hydrates, formes cristallines, et sels d'addition à un acide pharmaceutiquement acceptable et plus particulièrement son chlorhydrate, et le perindopril ou un de ses hydrates, formes cristallines et sels d'addition à une base pharmaceutiquement acceptable, et plus particulièrement ses sels d'arginine ou de tert-butylamine.

L'invention concerne également les compositions pharmaceutiques contenant l'association d'un inhibiteur sélectif et spécifique du courant I_{f} sinusal et d'un agent inhibiteur de l'enzyme de conversion de l'angiotensine, en combinaison avec un ou plusieurs excipients pharmaceutiquement acceptable,

Plus particulièrement, l'invention concerne les compositions pharmaceutiques contenant l'association d'un inhibiteur sélectif et spécifiques du courant I_{f} sinusal qui est l'ivabradine ainsi que ses hydrates, formes cristallines et sels d'addition à un acide pharmaceutiquement acceptable et plus particulièrement son chlorhydrate, et d'un agent inhibiteur de l'enzyme de conversion de l'angiotensine ou un de ses hydrates, formes cristallines, et sels d'addition à un acide pharmaceutiquement acceptable, en combinaison avec un ou plusieurs excipients pharmaceutiquement acceptable,

Préférentiellement, l'invention concerne les compositions pharmaceutiques contenant l'association d'un inhibiteur sélectif et spécifique du courant I_{f} sinusal qui est l'ivabradine ainsi que ses hydrates, formes cristallines et sels d'addition à un acide pharmaceutiquement acceptable et plus particulièrement son chlorhydrate, et d'un agent inhibiteur de l'enzyme de conversion de l'angiotensine qui est le périndopril ou un de ses hydrates, formes cristallines, et sels d'addition à une base pharmaceutiquement acceptable et plus particulièrement ses sels d'arginine ou de tert-butylamine, en combinaison avec un ou plusieurs excipients pharmaceutiquement acceptable.-,

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc... ainsi que les compositions pharmaceutiques avec libération programmée, retardée, prolongée ou différée.

Outre l'inhibiteur sélectif et spécifique du courant I_{f} sinusal et le composé inhibiteur de l'enzyme de conversion de l'angiotensine, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne de 1 à 500 mg d'ivabradine par 24 heures et plus préférentiellement 10 à 15 mg par jour et de manière encore préférée de 5 à 15mg par jour. La dose de l'agent inhibiteur de l'enzyme de conversion de l'angiotensine pourra être moindre que celle utilisée lorsqu'il est administré Seul.

Les exemples suivants illustrent l'invention et ne la limitent en aucune façon.

### Compositions pharmaceutiques :

### Formule de préparation pour 1000 comprimés dosés à 7,5 mg d'ivabradine et 2 mg de périndopril:

| | |
|---|---|
| Ivabradine, chlorhydrate | 7,5 g |
| Périndopril, *tert*-butylatuine | 2 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

D'autres exemples de compositions pharmaceutiques selon l'invention sont donnés ci-dessous, à titre non limitatif :

### Exemple 1

| Constituants | Quantité (mg) |
|---|---|
| ivabradine | 10 |
| périndopril, sel de tert-butytamiae | 4 |

### Exemple 2

| Constituants | Quantité (mg) |
|---|---|
| ivabradine | 10 |
| périndopril, sel de tert-butylamine | 8 |

### Exemple 3

| Constituants | Quantité (mg) |
|---|---|
| ivabradine | 15 |
| périndopril, sel de tert-butylamine | 4 |

### Exemple 4

| Constituants | Quantité (mg) |
|---|---|
| ivabradine | 15 |
| périndopril, sel de tert-butylamine | 8 |

### Exemple 5

| Constituants | Quantité (mg) |
|---|---|
| ivabradine | 10 |
| périndopril, sel d'arginine | 5 |

### Exemple 6

| Constituants | Quantité (mg) |
|---|---|
| ivabradine | 10 |
| périndopril, sel d'arginine | 10 |

### Exemple 7

| Constituants | Quantité (mg) |
|---|---|
| ivabradine | 15 |
| périndopril, sel d'arginine | 5 |

### Exemple 8

| Constituants | Quantité (mg) |
|---|---|
| ivabradine | 15 |
| périndopril, sel d'arginine | 10 |

La posologie pour les compositions pharmaceutiques décrites supra consiste en une administration per os d'un comprimé par 24 heures.

Dans les populations à risque correspondant à des patients âgés de plus de 75 ans, la dose critique initiale administrée par voie orale est de 5mg d'ivabradine et 2mg de périndopril, sel de tert-butylamine ou de 5mg d'ivabradine et 2.5mg de périndopril, sel d'arginine par 24 heures sous la forme d'un comprimé.

### Etude clinique n°1 :

Une étude clinique a été menée chez 8 patients présentant une hypertension artérielle qui étaient déjà traités avec un inhibiteur de l'enzyme de conversion (Périndopril n = 3, Ramipril n = 2, Enalapril n = 1, Lisinopril n = 1, Fosinopril n = 1) et une insuffisance cardiaque légère à modérée de classe 1 ou 2 selon la classification NYHA. Ces patients ont reçu de l'ivabradine à la dose de 7,5 mg 2 fois par jour pour 7 d'entre eux et de 5 mg 2 fois par jour pour 1 d'entre eux. Après 2 mois de traitement, durée reconnue comme suffisante pour atteindre l'effet maximum des deux traitements, les pressions artérielles systoliques et diastoliques moyennes allongées ont baissé de 7,5 mmHg et 7,3 mmHg respectivement. De plus si on considère les patients qui à l'inclusion dans cette étude avaient une pression artérielle qui n'était pas bien contrôlée sous inhibiteur de l'enzyme de conversion, c'est-à-dire ceux qui avaient encore une pression artérielle systolique ≥ 140 mmHg et/ou une pression artérielle diastolique ≥ 90 mmHg, la baisse de la pression artérielle dans ce groupe a été de 10 mmHg pour la pression artérielle systolique et de 8 mmHg pour la pression artérielle diastolique.

**Tableau 1 :**

| ***Evolution de la pression artérielle couchée à l'inclusion et après 2 mois de traitement avec un IEC et ivabradine*** | | |
|---|---|---|
| | Inclusion | 2 mois |
| PAS(mmHg) | 149,5 | 141,8 |
| PAD (mmHg) | 81,7 | 74,4 |

| | | |
|---|---|---|
| PAS : Pression artérielle systolique couchée PAD : Pression artérielle diastolique couchée | | |

**Tableau 2 :**

| ***Evolution de la pression artérielle couchée à l'inclusion et après 2 mois de traitement chez les patients qui présentaient une pression artérielle non contrôlée à l'entrée dans l'essai.*** | | |
|---|---|---|
| | Inclusion | 2 mois |
| PAS(mmHg) | 159 | 149 |
| PAD(mmHg) | 85 | 77 |

| | | |
|---|---|---|
| PAS : Pression artérielle systolique couchée PAD : Pression artérielle diastolique couchée | | |

Il a donc été observé une baisse substantielle de la pression artérielle lorsque l'on a ajouté l'ivabradine à un inhiteur de l'enzyme de conversion. Cette baisse de pression artérielle est inattendue car avec l'ivabradine la baisse moyenne qui est objectivée dans les essais cliniques est en général de l'ordre de 1 à 2 mmHg pour la pression artérielle diastolique et plutôt une légère augmentation de la pression artérielle systolique de l'ordre de 1 mmHg. Cette baisse est importante car on sait qu'une baisse de 4 à 5 mmHg chez les patients hypertendus diminue de façon très importante (30%) la survenue d'accidents cardiaques et neurologiques.

### Etude clinique n°2 :

Une étude clinique complémentaire a été réalisée chez des patients présentant une hypertension artérielle ainsi qu'une insuffisance cardiaque sévère de classe 3 selon la classification NYHA correspondant à une cardiopathie entraînant une limitation marquée de l'activité physique sans gêne au repos. Les patients ont été traités sur une durée de 6 semaines, durée reconnue comme suffisante pour observer clairement l'effet des traitements. Ces patients ont reçu par administration per os le traitement suivant:
- de l'ivabradine à la dose de 2.5mg 2 fois par jour pendant 2 semaines ; puis
- pendant les 2 semaines suivantes, de l'ivabradine à la dose de 5mg 2 fois par jour à l'exception des patients présentant une intolérance au produit, telle qu'une bradycardie excessive ou une symptomatologie clinique associée à une bradycardie marquée ; puis
- pendant les 2 dernières semaines, de l'ivabradine à la dose de 7.5mg 2 fois par jour sauf pour les patients présentant une intolérance au produit, telle qu'une bradycardie excessive ou une symptomatologie clinique associée à une bradycardie marquée.

40 patients déjà traités par un inhibiteur de l'enzyme de conversion tel que périndopril, ramipril, enalapril, lisinopril ou fosinopril ont reçu le traitement décrit supra. Les pressions artérielles systoliques et diastoliques moyennes allongées ont respectivement baissé de 2.5mmHg et 3.8mmHg.

**Tableau 3 :**

| ***Evolution de la pression artérielle couchée à l'inclusion et après 6 semaines de traitement avec un IEC et ivabradine*** | | |
|---|---|---|
| | Inclusion | 6 semaines |
| PAS (mmHg) | 126.3 | 123.8 |
| PAD (mmHg) | 78.4 | 74.6 |

| | | |
|---|---|---|
| PAS : Pression artérielle systolique couchée PAD : Pression artérielle diastolique couchée | | |

En outre, 11 patients, qui à l'inclusion dans cette étude avaient une pression artérielle qui n'était pas bien contrôlée sous inhibiteur de l'enzyme de conversion, c'est-à-dire ceux qui avaient encore une pression artérielle systolique ≥ 140mmHg et/ou une pression artérielle diastolique ≥ 90mmHg, ont reçu le traitement décrit supra. La baisse de la pression artérielle observée dans ce groupe a été de 10.7mmHg pour la pression artérielle systolique et de 8.6mmHg pour la pression artérielle diastolique.

**Tableau 4 :**

| ***Evolution de la pression artérielle couchée à l'inclusion et après 6 semaines de traitement avec un IEC et ivabradine, chez des patients présentant une pression artérielle non contrôlée à l'inclusion*** | | |
|---|---|---|
| | Inclusion | 6 semaines |
| PAS (mmHg) | 142.1 | 131.4 |
| PAD (mmHg) | 87.4 | 78.8 |

| | | |
|---|---|---|
| PAS : Pression artérielle systolique couchée PAD : Pression artérielle diastolique couchée | | |

Un groupe de 6 patients déjà traités avec périndopril comme inhibiteur spécifique de l'enzyme de conversion a reçu le traitement décrit supra. Une baisse significative de la pression artérielle a été observée à l'issue de 6 semaines de traitement, soit une baisse de 17.5mmHg de la pression artérielle systolique et de 7.7mmHg de la pression artérielle diastolique. Conformément aux conclusions de l'étude clinique précédente, cette baisse de la pression artérielle systolique et diastolique est considérée comme très importante quand on sait qu'une baisse de 4 à 5mmHg chez les patients hypertendus diminue de façon très importante (30%) la survenue d'accidents cardiaques et neurologiques.

**Tableau 5 :**

| ***Evolution de la pression artérielle couchée à l'inclusion et après 6 semaines de traitement avec périndopril et ivabradine*** | | |
|---|---|---|
| | Inclusion | 6 semaines |
| PAS (mmHg) | 127 | 109.5 |
| PAD (mmHg) | 77.5 | 69.8 |

| | | |
|---|---|---|
| PAS : Pression artérielle systolique couchée PAD : Pression artérielle diastolique couchée | | |

## Revendications

1. Association d'un inhibiteur sélectif et spécifique du courant I_{f} sinusal et d'un agent inhibiteur de l'enzyme de conversion de l'angiotensine.

2. Association selon la revendication 1 dans laquelle l'inhibiteur sélectif et spécifique du courant I_{f} sinusal est l'ivabradine ou 3-{3-[{[(7S) -3,4-diméthoxybicyclo[4.2.0]octa-1,3,5-trién-7-yl]méthyl}(méthyl)amino]propyl}-7,8-di-méthoxy-1,3,4,5-tétrahydro-2*H*-3-benzazépin-2-one, ou un de ses hydrates, formes cristallines ou sels d'addition à un acide pharmaceutiquement acceptable.

3. Association selon la revendication 1 dans laquelle l'inhibiteur sélectif et spécifique du courant I_{f} sinusal est l'ivabradine, chlorhydrate ou un de ses hydrates ou formes cristallines.

4. Association selon la revendication 1 dans laquelle l'agent inhibiteur de l'enzyme de conversion de l'angiotensine est le périndopril, ou un de ses hydrates, formes cristallines ou sels d'addition à une base pharmaceutiquement acceptable.

5. Association selon la revendication 1 dans laquelle l'agent inhibiteur de l'enzyme de conversion de l'angiotensine est le périndopril, sels de *tert*-butylamine ou d'arginine, ou un de leurs hydrates ou formes cristallines.

6. Association selon la revendication 1 dans laquelle l'inhibiteur sélectif et spécifique du courant I_{f} sinusal est l'ivabradine, chlorhydrate ou un de ses hydrates ou formes cristallines, et l'agent inhibiteur de l'enzyme de conversion de l'angiotensine est le périndopril, sels de *tert*-butylamine ou d'arginine, ou un de leurs hydrates ou formes cristallines.

7. Compositions pharmaceutiques contenant comme principe actif un inhibiteur sélectif et spécifique du courant I_{f} sinusal en association avec un agent inhibiteur de l'enzyme de conversion selon l'une des revendications 1 à 6, seuls ou en combinaison avec un ou plusieurs excipients pharmaceutiquement acceptables.

8. Compositions pharmaceutiques selon la revendication 7 contenant comme principe actif l'ivabradine, chlorhydrate ou un de ses hydrates ou formes cristallines, et le périndopril, sels de *tert*-butylamine ou d'arginine, ou un de leurs hydrates ou formes cristallines.

9. Compositions pharmaceutiques selon l'une des revendication 7 ou 8 utiles pour la fabrication d'un médicament pour le traitement de l'hypertension artérielle.

10. Utilisation d'une association selon l'une des revendications 1 à 6 pour l'obtention de compositions pharmaceutiques destinées au traitement de l'hypertension artérielle.
